# EUROPÄISCHE PATENTANMELDUNG

(11) **EP 4 656 168 A1**
(43) Veröffentlichungstag der Anmeldung: **03.12.2025**
(21) Anmeldenummer: 25175692.0
(22) Anmeldetag: 12.05.2025
(51) Int. Cl.: A61F 13/14, A41C 1/00, A41C 1/08, A61F 5/03

(54) **KOMPRESSIONSHOSE**

(30) Priorität: 29.05.2024 DE 102024115127
(71) Anmelder: Julius Zorn GmbH, 86551 Aichach (DE)
(72) Erfinder: TAEGER, Christian, 81479 München (DE); LEITLOFF, Mathias, 95463 Bindlach (DE)
(74) Vertreter: Charrier Rapp & Liebau

(57) **Zusammenfassung**

Die Erfindung betrifft eine Kompressionshose, insbesondere zur kompressiven Behandlung von Ödemen oder anderer Erkrankungen oder Verletzungen im Genital- oder Beckenbereich eines Patienten, umfassend ein Kompressionselement (1) zur Ausübung einer Kompression auf den Genitalbereich und/oder Beckenbereich des Patienten, ein Hosenteil (2) und mindestens ein am Kompressionselement unlösbar befestigtes oder angeformtes Taillenband (3), wobei das mindestens eine Taillenband (3) mittels Verschlusselemente (4) verstellbar im Bereich der Taille oder im oberen Hüftbereich des Patienten anlegbar ist, und das Hosenteil (2) im angezogenen Zustand zumindest den Beckenbereich des Patienten vollumfänglich umschließt. Um eine individuelle und präzise Anpassung an die Körperform, die Einstellung eines gewünschten, individuell und präzise anpassbaren Kompressionsdrucks sowohl im Genitalbereich und der Leistengegend als auch im Hüftbereich und ein bequemes Tragen über eine längere Behandlungsdauer zu ermöglichen, ist vorgesehen, dass das Kompressionselement (1) an dem Hosenteil (2) unlösbar befestigt ist.

## Beschreibung

Die Erfindung betrifft eine Kompressionshose, insbesondere zur kompressiven Behandlung von Ödemen oder anderer Erkrankungen oder Verletzungen im Genital- oder Beckenbereich eines Patienten oder zur Unterstützung der postpartalen Rückbildung nach einer Entbindung, nach dem Oberbegriff des Anspruchs 1.

Aus der EP 2 884 862 B1 ist ein medizinisches Kleidungsstück in Form einer Hüft- und Bauchstütze zur Unterstützung des Beckens und des Unterleibs eines Patienten bekannt, mit dem Schmerzen, Ödeme und Infektionen behandelt und kontrolliert werden können, die bei akuten oder chronischen Erkrankungen wie Krebs oder nach genitalen oder abdominalen Operationen oder vaginalen Entbindungen auftreten, um Komplikationen zu verringern und die Genesungszeit zu verbessern. Die Hüft- und Bauchstütze umfasst dabei ein die Taille umgebendes Element, das an seinem vorderen Teil verstellbar befestigt werden kann und einen oberen und einen unteren Rand sowie einen hinteren Teil aufweist, sowie Shorts, die nicht abnehmbar an der Unterkante des die Taille umschließenden Elements befestigt sind und einen Schrittbereich sowie ein Riemenelement aufweisen, wobei das Riemenelement an einem Ende am hinteren Teil des die Taille umgebenden Elements befestigt ist und dessen anderes Ende nach dem Hindurchführen unter dem Schrittbereich der Shorts am anderen Ende der Vorderseite des die Taille umgebenden Elements über einen Klettverschluss fixierbar ist, sodass das Riemenelement einen Druck auf den Schrittbereich des Trägers ausübt.

Die bekannte Hüft- und Bauchstütze stellt ein einstückiges und individuell einstellbares medizinisches Kleidungsstück dar, das unmittelbar nach einer Operation oder nach einer Entbindung und danach während einer mehrwöchigen Erholungsphase der zu behandelnden Person über der Unterwäsche getragen werden kann, um den Rücken, die Bauchmuskeln und die Vulva zu stützen. Das Anlegen des medizinischen Kleidungsstücks, insbesondere das Befestigen der Riemenelemente durch das Hindurchführen der Riemenelemente unter dem Schrittbereich erweist sich als umständlich und je nach Art der Beeinträchtigung des Trägers als schmerzhaft. Das bekannte Kleidungsstück erweist sich zudem als nachteilig hinsichtlich der Flexibilität und der individuellen Anpassung an verschiedene Patienten mit unterschiedlichen Größenverhältnissen im Bauch- und Hüftbereich. Weitere Nachteile des medizinischen Kleidungsstücks in Bezug auf den Tragekomfort ergeben sich aus der vollumfänglichen Befestigung der Shorts an das die Taille umgebende Element, bspw. beim Bücken, weil das die Taille umgebende Element dabei nach unten gezogen wird.

In anderen Anwendungsfällen für medizinische Hüft- und Bauchstützen, wie z.B. der kompressiven Behandlung von Genitalödemen oder anderen Ödemen im Becken- oder Leistenbereich, ist es darüber hinaus vorteilhaft, wenn ein vorgegebener Kompressionsdruck im Hüftbereich erzeugt werden kann. Hierfür ist die bekannte Hüft- und Bauchstütze nicht geeignet. Weiterhin ist bei der bekannten Hüft- und Bauchstütze eine individuelle Anpassung des ausgeübten Kompressionsdrucks an das Krankheitsbild des Patienten oder den Heilungsverlauf kaum möglich. Darüber hinaus erweist sich die bekannte Hüft- und Bauchstütze als unbequem, wenn ein erhöhter Kompressionsdruck auf den Genitalbereich mit den drei Riemen des Riemenelements eingestellt werden soll, weil sich die schmalen Riemen in den Genitalien einschnüren können. Insbesondere kann wegen der schmalen Ausbildung der einzelnen Riemenelemente mit der bekannten Hüft- und Bauchstütze kein flächiger und gleichförmiger Kompressionsdruck auf den Genitalbereich erzeugt werden. Das korrekte Einstellen der Riemenelemente erweist sich bei der bekannten Hüft- und Bauchstütze als schwierig. Fehlerhaft positionierte Riemenelemente können den Tragekomfort erheblich beeinflussen und darüber hinaus zu einer ungleichmäßigen Druckverteilung führen und Schmerzen verursachen.

Vor diesem Hintergrund besteht die Aufgabe der Erfindung in der Bereitstellung einer Kompressionshose zur kompressiven Behandlung von Ödemen oder anderer Erkrankungen oder Verletzungen im Genital- oder Beckenbereich eines Patienten oder einer Patientin, die eine individuelle und präzise Anpassung an die Körperform, die Einstellung eines gewünschten, individuell und präzise anpassbaren Kompressionsdrucks sowohl im Genitalbereich und der Leistengegend als auch im Hüftbereich und ein bequemes Tragen über eine längere Behandlungsdauer ermöglicht. Dabei soll der von der Kompressionshose erzeugte Kompressionsdruck auf einfache Weise während der Behandlungsdauer mit einer genauen Justierung des gewünschten Kompressionsdrucks präzise und auf einfache Weise einstellbar und an den Behandlungserfolg anpassbar sein.

Diese Aufgaben werden mit einer Kompressionshose mit den Merkmalen des Anspruchs 1 gelöst. Bevorzugte Ausführungsformen der Kompressionshose gehen aus den abhängigen Ansprüchen hervor.

Die erfindungsgemäße Kompressionshose dient insbesondere zur kompressiven Behandlung von Ödemen oder anderer Erkrankungen oder Verletzungen im Genital- oder Beckenbereich eines Patienten und kann insbesondere von männlichen und auch von weiblichen Patienten, bspw. zur postpartalen Rückbildung nach einer Entbindung, getragen werden. Die erfindungsgemäße Kompressionshose umfasst ein Kompressionselement zur Ausübung einer Kompression auf den Genitalbereich und/oder Beckenbereich des Patienten, ein Hosenteil und mindestens ein am Kompressionselement unlösbar befestigtes oder angeformtes Taillenband, wobei das mindestens eine Taillenband mittels Verschlusselemente verstellbar im Bereich der Taille oder im oberen Hüftbereich des Patienten anlegbar ist, und das Hosenteil im angezogenen Zustand zumindest den Beckenbereich des Patienten vollumfänglich umschließt. Dabei ist das Kompressionselement an dem Hosenteil unlösbar befestigt.

Unter unlösbarer Befestigung wird hier verstanden, dass zwei unlösbar miteinander verbundene Teile (wie z.B. das am Kompressionselement befestigte Taillenband und das an dem Hosenteil befestigte Kompressionselement) zumindest nicht ohne Werkzeuge oder Hilfsmittel voneinander getrennt werden können. Unlösbare Verbindungen in diesem Sinne können insbesondere durch Nähte, Verklebungen oder Verschweißungen oder auch durch einstückige, speziell materialeinstückige, Ausbildung der beiden Teile wie eine einstückige (insbesondere eine materialeinstückige) Anformung des einen Teils an dem anderen Teil, erzeugt werden. Unter einer lösbaren Befestigung wird demgegenüber verstanden, dass die beiden lösbar miteinander verbundenen Teile manuell und ohne Zuhilfenahme von Werkzeugen oder Hilfsmittel voneinander getrennt werden können. Lösbare Verbindungen können insbesondere durch Klettverschlüsse, Haftverschlüsse, Druckknopf-Verschlüsse oder Gürtelschnallen bereitgestellt werden.

Durch die unlösbare Befestigung des Kompressionselementes an dem Hosenteil entsteht eine einteilige Kompressionshose, die eine einfachere und schnellere Anpassung des Kompressionsdrucks ermöglicht als die aus dem Stand der Technik bekannte Kompressionshose mit Riemenelementen. Insbesondere kann der Patient bei der erfindungsgemäßen Kompressionshose selbst auf einfache Weise eine Anpassung des Kompressionsdrucks vornehmen, indem die Position des Taillenbands im Bereich der Taille oder im oberen Hüftbereich des Patienten variiert wird. Die unlösbare Befestigung des Kompressionselements an dem Hosenteil ermöglicht eine einfache, schnelle, gezielte und präzise Einstellung eines vom Kompressionselement auf den Genitalbereich bzw. den Leistenbereich des Patienten auszuübenden Kompressionsdrucks, wobei der ausgeübte Kompressionsdruck von der Anlegeposition des Hosenteils und des Taillenbands am Körper des Patienten, abhängt.

In einer bevorzugten Ausführungsform weist das mindestens eine Taillenband erste Befestigungsmittel auf und das Hosenteil enthält in einem Bundbereich zweite Befestigungsmittel, wobei das Hosenteil mittels einer lösbaren Kopplung der ersten Befestigungsmittel und der zweiten Befestigungsmittel an dem Taillenband abnehmbar befestigt werden kann.

Unter lösbarer Kopplung wird dabei, wie oben bereits erläutert, verstanden, dass die beiden lösbar miteinander verbundenen Teile (hier z.B. das Hosenteil an dem Taillenband) manuell und ohne Zuhilfenahme von Werkzeugen oder Hilfsmittel wieder voneinander getrennt werden können. Lösbare Verbindungen können insbesondere durch Klett- oder Haftverschlüsse oder durch Knopf- oder Druckknopf-Verschlüsse bereitgestellt werden.

Die lösbare Kopplung der ersten Befestigungsmittel und der zweiten Befestigungsmittel zur Befestigung des Hosenteils an dem Taillenband ermöglicht eine gezielte und präzise Einstellung sowie eine Fixierung eines vom Kompressionselement auf den Genitalbereich bzw. den Leistenbereich des Patienten auszuübenden Kompressionsdrucks und verhindert ein Verrutschen des Hosenteils. Dabei sind die ersten Befestigungsmittel und die zweiten Befestigungsmittel zweckmäßig so ausgestaltet, dass sie an unterschiedlichen Stellen miteinander gekoppelt werden können, wobei der ausgeübte Kompressionsdruck von der Position, an der die ersten und zweiten Befestigungsmittel miteinander gekoppelt werden, abhängt.

Die ersten Befestigungsmittel und die zweiten Befestigungsmittel sind bevorzugt als jeweils miteinander korrespondierende Bestandteile eines zweiteiligen, reversiblen Verbindungsmittels, insbesondere eines Klettverschlusses, eines Haftverschlusses oder einer Druckknopfverbindung, ausgebildet.

Die ersten Befestigungsmittel sind bspw. als flächiges Flauschteil eines Klettverschlusses ausgebildet, welches auf einer Außenseite des Taillenbands angeordnet ist. Die zweiten Befestigungsmittel können als eine Mehrzahl von dazu korrespondierenden Hakenteilen eines Klettverschlusses ausgebildet und an einer Innenseite des Hosenteils angeordnet sein. Dadurch können die zweiten Befestigungsmittel (Hakenteile) an einer Vielzahl von möglichen Fixierposition an den ersten Befestigungsmitteln (Flauschteil) fixiert werden, um einen gewünschten Kompressionsdruck des Kompressionselements einzustellen und dauerhaft während einer Behandlungsdauer oder eines Zyklus einer Behandlungsdauer aufrecht zu erhalten. Je höher die Fixierposition der zweiten Befestigungsmittel (Hakenteile) am ersten Befestigungsmittel (Flauschteil) ist, desto mehr wird das elastische Kompressionselement gedehnt und desto höher ist der vom Kompressionselement auf den Genitalbereich und die Leistengegend des Patienten ausgeübte Kompressionsdruck.

Das Kompressionselement ist bevorzugt als einteiliger Beutel oder als einteilige Schürze aus einem elastischen Textilmaterial ausgebildet. Durch die Dehnung des Kompressionselements wird bei einer am Patienten angelegten Kompressionshose ein gleichmäßiger, flächiger Kompressionsdruck erzeugt. Daraus ergibt sich gegenüber den aus dem Stand der Technik bekannten Kompressionshosen ein erhöhter Tragekomfort, sowie eine bessere und gleichmäßigere Druckverteilung.

Die erfindungsgemäße Kompressionshose ist dadurch insbesondere dazu geeignet, ein Ödem im Rahmen einer komplexen physikalischen Entstauungstherapie bestmöglich zu entstauen und es so auf eine Erhaltungsphase vorzubereiten, in der die erneute Ansammlung von Flüssigkeit vermieden wird. Das als Beutel bzw. Schürze ausgeformte Kompressionselement ist dabei zweckmäßig so dimensioniert, dass das Kompressionselement den Genitalbereich des Patienten zumindest weitgehend oder bevorzugt vollständig umschließt. Dies stellt sicher, dass auf den gesamten Ödembereich ein gleichmäßiger Kompressionsdruck ausgeübt wird.

Durch die bevorzugte beutelförmige oder schürzenförmige Ausbildung des Kompressionselements kann das Kompressionselement zweckmäßig eine an den Patienten angepasste anatomische Passform aufweisen, welche das Ödem im Genitalbereich des Patienten bevorzugt vollumfänglich umfasst. Dadurch wird einerseits der Tragekomfort verbessert und andererseits das Ausüben eines gleichmäßigen, flächigen Kompressionsdrucks auf das Ödem ermöglicht.

In einer Ausführungsform der Kompressionshose für Männer ist das beutelförmig ausgebildete Kompressionselement an die Anatomie des Patienten so angepasst, dass die Genitalien vollständig vom Kompressionselement aufgenommen werden können. Dies ermöglicht ein Einfassen eines zu behandelnden Ödems durch das Kompressionselement. Das beutelförmig ausgebildete Kompressionselement wirkt dabei auch als Stützbeutel für die Genitalien.

Das Taillenband ist mittels der am Taillenband angeordneten Verschlusselemente im Bereich der Taille oder des oberen Hüftbereichs des Patienten lösbar befestigbar. Die Verschlusselemente sind bevorzugt in einer Umfangsrichtung des Patienten an unterschiedlichen Positionen fixierbar. Dies ermöglicht das Anpassen des Taillenbandes auf die individuellen Größenverhältnisse des Patienten und eine präzise Einstellung eines durch das Taillenband auf den Taillenbereich und/oder den Hüftbereich des Patienten ausgeübten Drucks.

Die Verschlusselemente des Taillenbands sind bevorzugt als lösbare Befestigungselemente, insbesondere als Klettverschluss, Hakenverschluss, Druckknopfverbindung oder als Gürtelschnalle, ausgebildet und bevorzugt in einem Endabschnitt des Taillenbands angeordnet. Dadurch kann das Taillenband unter leichter Dehnung bequem und gleichzeitig sicher um den Taillen- oder Hüftumfang des Patienten angelegt und fixiert werden. Die Verschlusselemente sind dabei bevorzugt im Bauchbereich des Patienten angeordnet, wodurch dem Patienten ein bequemes Anlegen und Fixieren des Taillenbands ermöglicht wird. Durch Festlegen der Verschlusselemente an unterschiedlichen Positionen in einer Umfangsrichtung des Patienten kann ein vom Taillenband auf den Taillenbereich und/oder den Hüftbereich des Patienten ausgeübter Druck eingestellt werden.

In einer bevorzugten Ausführungsform der Kompressionshose besteht das Taillenband aus einem ersten Teilband und einem zweiten Teilband, die jeweils unlösbar am Kompressionselement befestigt sind und die mittels der Verschlusselemente, insbesondere eines Klettverschlusses oder einer Gürtelschnalle, miteinander verbindbar sind und dabei insbesondere variabel in der Umfangsrichtung des Patienten einstellbar sind. Durch ein Taillenband, das aus zwei Teilbändern besteht, lässt sich das Taillenband leichter anlegen und ein Kompressionsdruck, der auf den Bauch des Patienten ausgeübt wird, kann leichter und präziser eingestellt werden. Gleichzeitig kann ein Verrutschen des Kompressionselementes vermieden werden.

In einer bevorzugten Ausführungsform der Kompressionshose ist das Kompressionselement an einem ersten Ende mit einer nicht lösbaren Verbindung, insbesondere mit einer ersten Naht, an dem Hosenteil befestigt. Die nicht lösbare Verbindung ist dabei insbesondere in einem Schrittbereich, welcher bei angezogenem Hosenteil im Schritt des Patienten zu liegen kommt, an einer Innenseite des Hosenteils angeordnet. Durch die unlösbare Verbindung des Kompressionselements am Hosenteil in einem Schrittbereich wird gewährleistet, dass das Kompressionselement richtig sitzt und ein gleichmäßiger, flächiger Kompressionsdruck an der richtigen Stelle ausgeübt wird. Die Gefahr einer ungünstigen Druckverteilung durch fehlerhaftes anlegen der Kompressionshose durch den Patienten wird durch die unlösbare Befestigung des Kompressionselements an dem Hosenteil minimiert.

Unter unlösbarer Befestigung wird dabei, wie oben bereits erläutert, verstanden, dass die beiden unlösbar miteinander verbundenen Teile (hier z.B. das Kompressionselement an dem Hosenteil) zumindest nicht ohne Werkzeuge oder Hilfsmittel voneinander getrennt werden können.

Das Taillenband der Kompressionshose ist mit einer unlösbaren Verbindung, insbesondere mit einer zweiten Naht, an dem Kompressionselement befestigt, sodass für Taillenband und Kompressionselement unterschiedliche Materialien mit auf die Anwendung abgestimmten Eigenschaften verwendet werden können. Die Verbindung kommt bei angelegtem Taillenband insbesondere in einem Bauchbereich oder Beckenbereich zu liegen. Bei der Ausführungsform mit zwei Teilbändern sind beide Teilbänder mit unlösbaren Verbindungen, insbesondere mit zweiten Nähten an dem Kompressionselement befestigt, wobei die Verbindungen im angelegten Zustand der Kompressionshose vorzugsweise und zumindest im Wesentlichen senkrecht zur Umfangsrichtung seitlich am Kompressionselement angeordnet sind.

Eine unlösbare Verbindung zwischen Taillenband und Kompressionselement kann auch in Form eines am Kompressionselement angeformten Taillenbands bestehen. Bei am Kompressionselement angeformtem Taillenband sind das Taillenband und das Kompressionselement einstückig und speziell materialeinstückig, bspw. aus einem Stück eines elastischen Textilmaterials, hergestellt, insbesondere aus einem elastischen Kompressionsgestrick, und weisen keine Naht auf. Eine einstückige Anformung des Taillenbands am Kompressionselement kann bspw. durch nahtloses Anstricken des Kompressionselements am Taillenband oder durch Ausformung des Kompressionselements und des einstückig daran angeformten Taillenbands mittels Zuschneiden aus einem flächigen Textilmaterial erfolgen. Ein Vorteil einer nahtlosen bzw. angeformten Variante besteht in einem höheren Tragekomfort, da lokale Druck- und Reibungsstellen aufgrund von Nähten bzw. Nahtzugaben auf der Innenseite der Kompressionshose vermieden werden.

Wenn die Kompressionshose am Patienten angelegt ist, befindet sich das Kompressionselement bevorzugt innerhalb des Hosenteils. Das Kompressionselement wird dabei vom dem Hosenteil teilweise oder vollständig überdeckt. Dadurch werden Druckstellen aufgrund eines Faltenwurfes eines unter dem Kompressionselement liegenden Hosenteils vermieden. Ein das Kompressionselement verdeckendes Hosenteil ist zudem aus ästhetischen Gründen vorteilhaft.

Das Taillenband wird bevorzugt zumindest teilweise oder vollständig vom Hosenteil verdeckt wenn die Kompressionshose am Patienten angelegt ist. Eine bereichsweise Überlappung eines oberen Abschnitts des Hosenteils, insbesondere eines Bundabschnitts des Hosenteils, mit dem darunter angeordneten Taillenband, ist insbesondere in Bezug auf ein bequemes Tragen der Kompressionshose vorteilhaft. Es ist jedoch möglich, dass das Hosenteil vollständig unterhalb und im Abstand zum Taillenband am Patienten angeordnet wird und der Bundbereich des Hosenteils das Taillenband nicht überlappt. Ein zumindest geringfügig unter dem Hosenteil herausragendes Taillenband ist durchaus vorteilhaft, weil dadurch das Taillenband manuell ergriffen werden kann, um bspw. durch Veränderung der Kopplungsposition der Verschlusselemente oder durch Verändern der Anlegeposition des Taillenbandes den vom Kompressionselement ausgeübten Kompressionsdruck einstellen, justieren oder nachstellen zu können.

Bei an einem Patienten angelegter Kompressionshose erfährt das insbesondere aus einem elastischen Textilmaterial gebildete Kompressionselement eine von der Anlegeposition des Taillenbandes und eines Bundbereiches des Hosenteils abhängige Dehnung. Das Kompressionselement übt aufgrund der Dehnung einen vom Dehnungsgrad abhängigen Kompressionsdruck auf den Genitalbereich und die Leistengegend des Patienten aus. Der Kompressionsdruck ist dabei durch Auswahl der Anlegepositionen einstellbar und kann auch nach Anlegen der Kompressionshose am Patienten noch geändert werden, um bspw. den Kompressionsdruck im Verlauf einer längeren Behandlungsperiode anpassen zu können. Zur Anpassung des Kompressionsdrucks ist dabei nur eine Änderung der Anlegeposition des Taillenbandes und/oder des Hosenteils erforderlich, ohne dass die gesamte Kompressionshose abgenommen werden muss.

Sowohl das Hosenteil als auch das Kompressionselement sind zweckmäßig jeweils aus einem elastischen Textilmaterial gefertigt. Bevorzugt ist das Hosenteil aus einem Kompressionsgestrick oder einem elastisch gewebten Textilmaterial gebildet. Auch das Kompressionselement kann aus einem Kompressionsgestrick oder einem gewebten Textilmaterial gebildet sein. Das Taillenband ist bevorzugt aus einem beidseitig mit einem Veloursmaterial kaschierten Schaumstoff gebildet. Die Dehnbarkeit des Taillenbands ist bevorzugt geringer als die Dehnbarkeit des Hosenteils und des Kompressionselements.

Das Hosenteil der Kompressionshose ist bevorzugt ein Kompressionstextil aus einem elastischen Textilmaterial und ist insbesondere aus einem elastischen Kompressionsgestrick hergestellt, wobei das Textilmaterial bevorzugt so ausgewählt und das Hosenteil so dimensioniert ist, dass das Hosenteil, insbesondere im Hüft- und Beckenbereich, einen vorgegebenen Kompressionsdruck auf den Patienten ausübt. Besonders bevorzugt ist das Hosenteil aus einem elastischen Kompressionsgestrick mit einem Grundgestrick und einem darin eingelegten elastischen Schussfaden gefertigt. Dies ermöglicht die Einstellung eines gewünschten Kompressionsdrucks über die Auswahl und die Dehnung des Schussfadenmaterials und die Anpassung der Dimensionierung des Hosenteils auf die Anatomie des Patienten, insbesondere durch eine Maßanfertigung. Das Hosenteil kann auch aus einer rundgestrickten Meterware eines elastischen Textilmaterials gefertigt sein.

Durch die Ausbildung des Hosenteils als ein Kompressionstextil wird zusätzlich zu dem vom Kompressionselement auf den Genitalbereich des Patienten ausgeübten Druck auch auf den Hüftbereich und/oder den Beckenbereich des Patienten ein vollumfänglich wirkender Kompressionsdruck erzeugt. Dadurch können Ödeme, die sich vom Genitalbereich über die Leistengegend bis in den Hüft- und Beckenbereich erstrecken, wirksam durch eine Kompressionstherapie behandelt werden.

Um die Anlegeposition des Hosenteils auf einfache Weise fixieren zu können, kann das Hosenteil in einer weiteren Ausführungsform im Bundbereich einen elastischen Bund und/oder einen Tunnelbund aufweisen. Weiterhin können zur Verbesserung des Sitzes der Kompressionshose am Patienten an dem Hosenteil und/oder am Taillenband Hosenträger angeordnet sein.

In einer bevorzugten Ausführungsform ist eine an der Innenseite des Kompressionselements, die bei angelegter Kompressionshose zum Körper des Patienten weist, einlegbare und an das Kompressionselement lösbar, bspw. über einen Klettverschluss, anbringbare Pelotte vorgesehen, die zur Erhöhung des vom Kompressionselement auf ein Ödem ausgeübten Drucks dient und zweckmäßig eine an die Körperform der zu behandelnden Person angepasste Form hat. Insbesondere können hierbei Pelotten mit einer für Männer oder Frauen geeigneten Passform im Schritt- bzw. Dammbereich eingesetzt werden. Ergänzend oder anstelle einer Befestigung der Pelotte an der Innenseite des Kompressionselements mittels eines lösbaren Befestigungsmittels, wie z.B. eines Klettverschlusses, kann an dem Kompressionselement, insbesondere an seiner Innenseite, auch eine Tasche vorgesehen sein, in die die Pelotte herausnehmbar eingelegt werden kann.

Die erfindungsgemäße Kompressionshose ermöglicht daher insgesamt eine individuelle und präzise Einstellung eines von der Kompressionshose auf ein Genital-Ödem bzw. auf den Unterbauch und den Hüftbereich des Patienten ausgeübten Kompressionsdrucks sowie einen verbesserten Tragekomfort. Durch das über das Kompressionselement mit dem Hosenteil verbundene Taillenband entsteht eine einteilige Kompressionshose, die eine einfache und schnelle Anpassung des Kompressionsdrucks ermöglicht, um ein Ödem effektiv behandeln zu können. Gleichzeitig gewährleistet die erfindungsgemäße Kompressionshose eine hohe Flexibilität in der Höhenverstellung bei verschiedenen Körperumfängen einer zu behandelnden Person.

Diese und weitere Vorteile sowie bevorzugte Merkmale der Erfindung ergeben sich aus dem nachfolgend unter Bezugnahme auf die Zeichnungen beschriebenen Ausführungsbeispiel. Dabei zeigen
- **Fig. 1:**: Detailansicht der Innenseite einer Kompressionshose mit einem Taillenband bestehend aus zwei Teilbändern, einem Hosenteil und einem innerhalb des Hosenteils liegenden und im Schrittbereich des Hosenteils befestigten Kompressionselement, wobei die Innenseite des in dem Hosenteil liegenden Kompressionselements gezeigt ist;
- **Fig. 2:**: Darstellung der Kompressionshose von Figur 1 in einem an einem Patienten angelegten Zustand in einer Vorderansicht;
- **Fig. 3:**: Darstellung der Kompressionshose von Figur 2 in einem am Patienten angelegten Zustand in einer Rückansicht;
- **Fig. 4:**: Darstellung der Kompressionshose von Figur 1 in einer Vorderansicht mit heruntergezogenem Hosenteil zur besseren Darstellung des Kompressionselements und des Taillenbands der Kompressionshose, wobei der Bundbereich des Hosenteils nach außen umgestülpt ist;
- **Fig. 5:**: Darstellung der Kompressionshose von Figur 4 mit heruntergezogenem Hosenteil in einer Rückansicht, wobei der Bundbereich des Hosenteils nach außen umgestülpt ist;

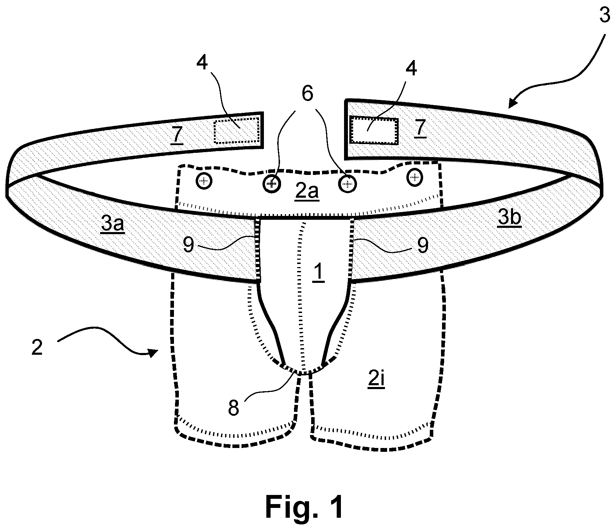

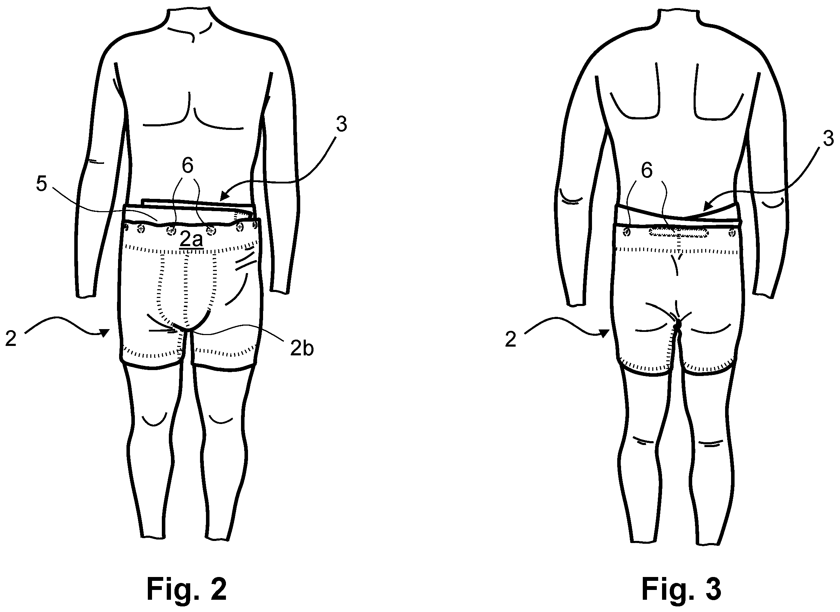

Die in Figur 1 dargestellte Ausführungsform einer Kompressionshose, welche insbesondere zur kompressiven Behandlung von Ödemen oder anderer Erkrankungen oder Verletzungen im Genital- oder Beckenbereich eines Patienten eingesetzt werden kann, umfasst ein Taillenband 3 mit Teilbändern 3a und 3b, ein an den Teilbändern unlösbar befestigtes Kompressionselement 1 und ein an dem Kompressionselement 1 unlösbar befestigtes Hosenteil 2. Die Darstellung der Figur 1 zeigt die umgestülpte Kompressionshose in einer Innenansicht, sodass die Innenseiten 2i des Hosenteils 2 und des Kompressionselements 1 gezeigt werden. Die gestrichelte Darstellung des Hosenteils 2 verdeutlicht, dass Bereiche des Hosenteils 2 durch das Kompressionselement 1 und die Teilbänder 3a und 3b des Taillenbands 3 überdeckt werden. Im angezogenen Zustand der dargestellten Ausführungsform der Kompressionshose, der insbesondere in Figur 2 und Figur 3 gezeigt ist, liegen das Kompressionselement 1 und das Taillenband 3 innerhalb des Hosenteils 2 und werden ganz oder zumindest teilweise von diesem verdeckt.

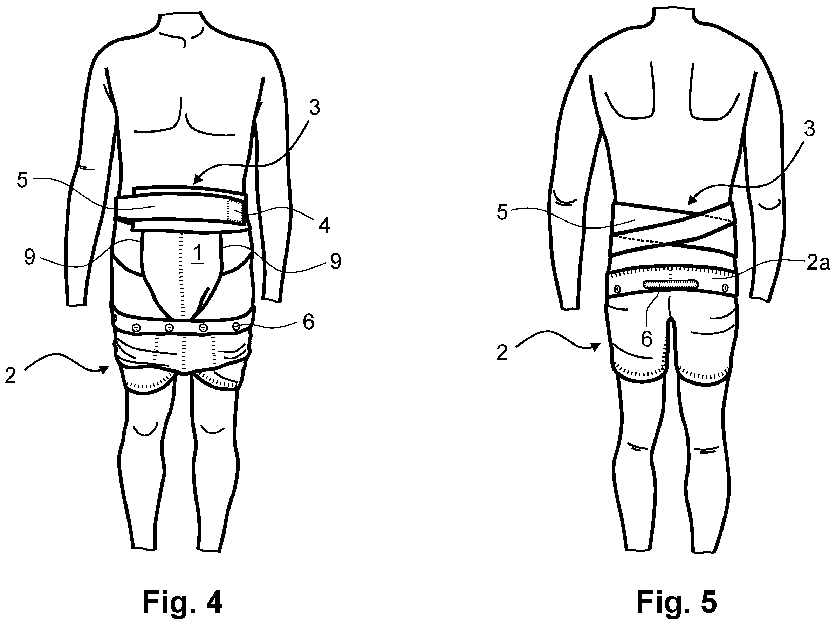

In der dargestellten Ausführungsform besteht das Taillenband 3 aus einem ersten Teilband 3a und einem zweiten Teilband 3b, die jeweils an einem Ende mit einer zweiten Naht 9 unlösbar am Kompressionselement 1 befestigt sind. Die zweiten Nähte 9 kommen bei angelegtem Taillenband 3 insbesondere in einem Bauchbereich oder Beckenbereich des Patienten zu liegen. Die Außenseite des Taillenbands 3, die auch in Figur 4 und Figur 5 zu sehen ist, ist als flächiges Flauschteil eines Klettverschlusses ausgebildet, welches erste Befestigungsmittel 5 bildet.

In einem Endabschnitt 7 am anderen (freien) Ende der Teilbänder 3a und 3b sind jeweils lösbare Verschlusselemente 4 angeordnet, mittels derer die Teilbänder 3a und 3b miteinander verbindbar sind und dabei insbesondere variabel in der Umfangsrichtung des Patienten einstellbar sind. Die Teilbänder 3a, 3b können dabei auch mehrfach um den Umfang des Patienten geschlungen werden. Bei den lösbaren Verschlusselementen 4 kann es sich beispielsweise um ein Klettelement mit Wiederhaken eines Klettverschlusses, um einen Hakenverschluss oder um eine Gürtelschnalle handeln. Das Taillenband 3 kann mittels der Verschlusselemente 4 im Bereich der Taille des Patienten durch Kopplung der Verschlusselemente 4, beispielsweise durch Schließen einer Gürtelschnalle oder durch Verbindung des Hakenteils des Klettelements mit einem durch die Außenseite des Taillenbandes 3 gebildeten Flauschteil, fixiert werden. Die Fixierung des Taillenbands 3 ist beispielsweise der Darstellung von Figur 4 zu entnehmen. Durch die Positionierung der Fixierungsstelle der Verschlusselemente 4 kann dabei ein ausgewählter Druck, der vom Taillenband 3 auf die Taille des Patienten ausgeübt wird, eingestellt werden. Zum Abnehmen des Taillenbands 3 vom Patienten können die Verschlusselemente 4 gelöst werden, indem beispielsweise die Kopplung des Klettverschlusses gelöst oder die Gürtelschnalle geöffnet wird.

In der dargestellten Ausführungsform ist das Kompressionselement 1 mit den zweiten Nähten 9 an den Enden der Teilbänder 3a und 3b des Taillenbands 3 befestigt und kommt im angelegten Zustand der Kompressionshose im Genitalbereich und/oder Beckenbereich des Patienten zu liegen, was besonders aus Figur 4 ersichtlich ist. Die zweiten Nähte 9 sind dabei an seitlichen Kanten des Kompressionselements 1 in einem oberen Bereich angeordnet und kommen bei angelegter Kompressionshose insbesondere in einem Bauchbereich oder Beckenbereich des Patienten zu liegen. Das Kompressionselement 1 ist als einteiliger Beutel oder als einteilige Schürze aus einem elastischen Textilmaterial ausgebildet.

Das Kompressionselement 1 ist an einem ersten Ende mit einer nicht lösbaren Verbindung, insbesondere mit einer ersten Naht 8, an dem Hosenteil 2 befestigt (Figur 1). In der zeichnerisch dargestellten Ausführungsform verbindet die erste Naht 8 ein erstes Ende des Kompressionselements 1 mit einem Schrittbereich 2b an einer Innenseite des Hosenteils 2. Bei angezogener Kompressionshose kommt die erste Naht 8 im Schritt des Patienten zu liegen.

Figur 2 und Figur 3 zeigen die Kompressionshose in einer Vorderansicht und einer Rückansicht in einem an einem Patienten angelegten Zustand. Das Hosenteil 2 der Kompressionshose befindet sich dabei außen und in teilweiser Überlappung mit dem darunterliegenden Taillenband 3. Das Hosenteil 2 ist in dem zeichnerisch dargestellten Ausführungsbeispiel als Short mit einem schlauchförmigen Rumpfabschnitt, der mittig einen Schrittbereich 2b enthält, zwei am Rumpfabschnitt angeordnete schlauchförmige Beinabschnitte und einem am oberen Rand des Rumpfabschnitts angeordneten Bundbereich 2a ausgebildet. Die Länge der beiden Beinabschnitte ist dabei variabel, d.h. die Länge der Beinabschnitte kann auch länger oder kürzer sein als in dem hier zeichnerisch dargestellten Ausführungsbeispiel. Insbesondere kann das Hosenteil 2 auch als Strumpfhose mit Beinabschnitten ausgebildet sein, wobei sich die Beinabschnitte bis zum Knöchel des Patienten erstrecken können und optional auch ein Fußteil enthalten können.

In dem zeichnerisch dargestellten Ausführungsbeispiel sind an der Innenseite 2i das Hosenteils 2 in dem Bundbereich 2a lösbare zweite Befestigungsmittel 6, insbesondere in Form einer Mehrzahl von Hakenteilen eines Klettverschlusses oder Druckknöpfe, vorgesehen, wie insbesondere aus Figur 1, Figur 4 und Figur 5 hervorgeht. Figur 4 und Figur 5 zeigen die Kompressionshose in einem am Patienten angelegten Zustand in einer Vorderansicht und einer Rückansicht, wobei das Kompressionselement 1 und das Taillenband 3 durch ein teilweise heruntergezogenes Hosenteil 2 sichtbar werden und der Bundbereich 2a des Hosenteils nach außen umgeschlagen ist. Die zweiten Befestigungsmittel 6 dienen zur lösbaren Befestigung des Hosenteils 2 an den ersten Befestigungsmitteln 5, die an einer Außenseite des Taillenbands 3 vorgesehen sind. Bevorzugt sind die zweiten Befestigungsmittel 6 als ein Klettband mit Wiederhaken ausgebildet, die mit dem an der Außenseite des Taillenbands 3 angeordneten Klettpad (Flauschteil) eines Klettverschlusses zusammenwirken. Durch eine Kopplung der zweiten Befestigungsmittel 6 des Hosenteils 2 mit den ersten Befestigungsmitteln 5 des Taillenbands 3 kann das Hosenteil 2 lösbar an einer ausgewählten Position am Taillenband 3 fixiert werden. Die Fixierung des Hosenteils 2 am Taillenband 3 im angezogenen Zustand der Kompressionshose ist beispielsweise den Darstellungen von Figur 2 und Figur 3 zu entnehmen.

Zum Anlegen der Kompressionshose an einem Patienten zieht der Patient zunächst das Hosenteil 2 mit innenliegendem Kompressionselement 1 und geöffnetem Taillenband 3 an, wobei das im Schrittbereich 2b des Hosenteils 2 angenähte Kompressionselement 1 im Schritt des Patienten zu liegen kommt. Wie in Figur 4 und Figur 5 dargestellt, bleibt der Bundbereich 2a des Hosenteils 2 dabei vorerst noch unterhalb des Hüftbereiches des Patienten und kann nach außen hin umgeklappt sein.

In diesem Zustand wird das Taillenband 3 um die Taille des Patienten gelegt und mittels der Verschlusselemente 4 fixiert. Durch Wahl der Verschlussposition der Verschlusselemente 4 kann dabei ein bestimmter Druck vom Taillenband 3 auf den Taillen- und Hüftbereich des Patienten ausgeübt werden.

Nach Anlegen des Taillenbands 3 wird das Hosenteil 2 nach oben gezogen, sodass sich das Kompressionselement 1 innerhalb des Hosenteils 2 befindet und zumindest im Wesentlichen vollständig vom Hosenteil 2 überdeckt wird. Wenn das Hosenteil 2 hochgezogen wird, wird die Anlegeposition des Bundbereich 2a des Hosenteils 2 gewählt. Bei Ausführungsformen, die an dem Hosenteil 2 angeordnete Hosenträger, einen elastischen Bund oder einen Tunnelbund aufweisen, kann eine Anlegeposition oberhalb oder unterhalb des Taillenbandes 3 gewählt werden.

Aufgrund der elastischen Eigenschaften des Kompressionselements 1, welches aus einem elastischen Textilmaterial gebildet ist, wird das Kompressionselement 1 je nach Anlegeposition des Taillenbandes 3 und des Bundbereiches 2a mehr oder weniger gedehnt. Durch Auswahl der Anlegeposition kann der Grad der Dehnung des Kompressionselements 1 und dadurch ein Kompressionsdruck, der vom Kompressionselement 1 auf den Genitalbereich und die Leistengegend des Patienten ausgeübt wird, eingestellt oder variiert werden.

Bei der in den Zeichnungen gezeigten Ausführungsform, kann das Hosenteil 2 durch Kopplung der im Bundbereich 2a angeordneten zweiten Befestigungsmittel 6 mit den ersten Befestigungsmitteln 5 am Taillenband 3 fixiert werden. Dabei kann der Kompressionsdruck, der vom Kompressionselement 1 auf den Genitalbereich und die Leistengegend des Patienten ausgeübt wird über die Fixierungsposition der ersten und der zweiten Befestigungsmittel 5 und 6 fest eingestellt werden. Die Fixierungsposition, an der die zweiten Befestigungsmittel 6 mit den flächig ausgebildeten ersten Befestigungsmitteln 5 gekoppelt sind, legt damit den vom Kompressionselement 1 auf den Genitalbereich des Patienten ausgeübten Kompressionsdruck fest.

In der in den Zeichnungen dargestellten bevorzugten Ausführungsform sind als zweite Befestigungsmittel 6 eine Mehrzahl von Hakenteilen eines Klettverschlusses oder Druckknöpfe vorgesehen, die an der Innenseite 2i des Hosenteils 2 im Bereich des Bunds 2a angeordnet sind. Mit diesen zweiten Befestigungsmitteln 6 kann die Innenseite 2i des Hosenteils 2, insbesondere im Bereich des Bunds 2a, auf der Außenseite des im Überlappungsbereich darunterliegenden Taillenbands 3 abnehmbar befestigt werden. Wenn die zweiten Befestigungsmittel 6 Druckknöpfe sind, enthält bspw. die Außenseite des Taillenbands 3 entsprechend der Anzahl der Druckknöpfe eine Mehrzahl von korrespondierenden Aufnahmen mit Vertiefungen, in die jeweils ein an der Innenseite des Bunds 2a des Hosenteils 2 angeordnetes Kopfteil eines Druckknopfs lösbar verankert werden kann, oder umgekehrt.

Zum Ausziehen der Kompressionshose wird der oben beschriebene Vorgang zum Anlegen der Kompressionshose an einem Patienten in umgekehrter Reihenfolge durchgeführt. Hierfür wird zunächst die Kopplung der ersten und zweiten Befestigungsmittel 5 und 6 gelöst und dann das das Hosenteil 2, wie in Figur 4 und Figur 5 gezeigt, nach unten geschoben. Anschließend werden die Verschlusselemente 4 des Taillenbands 3 geöffnet und die Kompressionshose kann mit dem integrierten Kompressionselement 1 ausgezogen werden.

Die Ausbildung der erfindungsgemäßen Kompressionshose in einteiliger Form ermöglicht dabei ein einfaches und bequemes An- und Ausziehen der Kompressionshose sowie ein schnelles und präzises Anpassen des Kompressionsdrucks auf den Genitalbereich und die Leistengegend des Patienten über die Einstellung der Dehnung des Kompressionselements 1. Der vom Kompressionselement 1 ausgeübte Kompressionsdruck kann dabei nachträglich, d.h. nach Anlegen der Kompressionshose am Patienten, einfach und schnell nachjustiert werden. Dies ist beispielsweise bei einer länger anhaltenden Kompressionstherapie von Vorteil, um den Kompressionsdruck an den Heilungsverlauf anpassen zu können. So ist es z.B. möglich, den Kompressionsdruck, je nach Heilungsverlauf, schrittweise während der Behandlungsdauer zu verringern.

Sowohl das Hosenteil 2 als auch das Kompressionselement 1 sind zweckmäßig jeweils aus einem elastischen Textilmaterial gefertigt. Bevorzugt ist das Hosenteil 2 aus einem Kompressionsgestrick mit einem Grundgestrick aus einem elastischen Faden und einem darin eingelegten bzw. eingestrickten elastischen Schussfaden gebildet. Das Hosenteil 2 und das Kompressionselement 1 können auch aus einer rundgestrickten Meterware gefertigt sein.

Insbesondere kann es sich bei dem Material des Hosenteils 2 um eine rundgestrickte Ware aus 80% Polyamid und 20% Elasthan handeln. Auch das Kompressionselement 1 kann aus einem Kompressionsgestrick gebildet sein. Das Kompressionselement 1 ist bevorzugt ein gewebtes Textilmaterial, beispielsweise aus 71% Polyamid und 31% Elasthan. Das Taillenband 3 ist bevorzugt aus einem beidseitig mit einem Velourmaterial kaschierten Schaumstoff gebildet, beispielsweise aus 74% Polyamid, 11% Polyurethan und 15% Elasthan. Die Dehnbarkeit des Taillenbands 3 ist dabei bevorzugt geringer als die Dehnbarkeit des Hosenteils 2 und des Kompressionselements 1.

## Patentansprüche

1. Kompressionshose, insbesondere zur kompressiven Behandlung von Ödemen oder anderer Erkrankungen oder Verletzungen im Genital- oder Beckenbereich eines Patienten, umfassend ein Kompressionselement (1) zur Ausübung einer Kompression auf den Genitalbereich und/oder Beckenbereich des Patienten, ein Hosenteil (2) und mindestens ein am Kompressionselement unlösbar befestigtes oder angeformtes Taillenband (3), wobei das mindestens eine Taillenband (3) mittels Verschlusselemente (4) verstellbar im Bereich der Taille oder im oberen Hüftbereich des Patienten anlegbar ist, und das Hosenteil (2) im angezogenen Zustand zumindest den Beckenbereich des Patienten vollumfänglich umschließt, **dadurch gekennzeichnet, dass** das Kompressionselement (1) an dem Hosenteil (2) unlösbar befestigt ist.

2. Kompressionshose nach Anspruch 1, wobei das mindestens eine Taillenband (3) erste Befestigungsmittel (5) aufweist und das Hosenteil (2) in einem Bundbereich (2a) zweite Befestigungsmittel (6) aufweist, wobei das Hosenteil (2) mittels einer lösbaren Kopplung der ersten Befestigungsmittel (5) und der zweiten Befestigungsmittel (6) an dem mindestens einen Taillenband (3) abnehmbar befestigt werden kann.

3. Kompressionshose nach Anspruch 2, wobei die ersten Befestigungsmittel (5) und die zweiten Befestigungsmittel (6) jeweils miteinander korrespondierende Bestandteile eines zweiteiligen, reversiblen Verbindungsmittels, insbesondere eines Klettverschlusses, eines Haftverschlusses oder einer Druckknopfverbindung sind.

4. Kompressionshose nach Anspruch 2 oder 3, wobei die ersten Befestigungsmittel (5) flächig und insbesondere als flächiges Flauschteil eines Klettverschlusses ausgebildet und auf einer Außenseite des Taillenbands (3) angeordnet sind, und wobei die zweiten Befestigungsmittel (6) insbesondere als eine Mehrzahl von Hakenteilen eines Klettverschlusses ausgebildet und an einer Innenseite (2i) des Hosenteils (2) angeordnet sind.

5. Kompressionshose nach einem der voranstehenden Ansprüche, wobei das Kompressionselement (1) als einteiliger Beutel oder als einteilige Schürze aus einem elastischen Textilmaterial ausgebildet ist.

6. Kompressionshose nach einem der voranstehenden Ansprüche, wobei die Verschlusselemente (4), mit denen das Taillenband (3) lösbar am Patienten, insbesondere in dessen Bauchbereich, befestigbar ist, die Einstellung eines durch das Taillenband (3) auf den Taillenbereich und/oder den Hüftbereich des Patienten ausgeübten Drucks ermöglichen, indem die Verschlusselemente (4) in einer Umfangsrichtung des Patienten an unterschiedlichen Positionen fixierbar sind.

7. Kompressionshose nach einem der voranstehenden Ansprüche, wobei das Taillenband (3) aus einem ersten Teilband (3a) und einem zweiten Teilband (3b) besteht, wobei das erste Teilband (3a) und das zweite Teilband (3b) unlösbar am Kompressionselement (1) befestigt sind und das erste Teilband (3a) und das zweite Teilband (3b) mittels der Verschlusselemente (4) miteinander verbindbar sind, insbesondere mit einer Gürtelschnalle.

8. Kompressionshose nach einem der voranstehenden Ansprüche, wobei die Verschlusselemente (4) als lösbare Befestigungselemente, insbesondere als Klettverschluss oder als Hakenverschluss, ausgebildet und zumindest in einem Endabschnitt (7) des Taillenbands (3) angeordnet sind.

9. Kompressionshose nach einem der voranstehenden Ansprüche, wobei das Kompressionselement (1) mit einer unlösbaren Verbindung, insbesondere mit einer ersten Naht (8), an dem Hosenteil (2) befestigt ist, wobei die Verbindung insbesondere in einem Schrittbereich (2b), welcher bei angezogenem Hosenteil (2) im Schritt des Patienten zu liegen kommt, angeordnet ist.

10. Kompressionshose nach einem der voranstehenden Ansprüche, wobei das mindestens eine Taillenband (3) mit einer unlösbaren Verbindung, insbesondere mit einer zweiten Naht (9), an dem Kompressionselement (1) befestigt ist, wobei die Verbindung bei angelegtem Taillenband (3) insbesondere in einem Bauchbereich oder Beckenbereich zu liegen kommt.

11. Kompressionshose nach einem der voranstehenden Ansprüche, wobei im angezogenen Zustand das Kompressionselement (1) bei am Patienten angelegter Kompressionshose innerhalb des Hosenteils (2) liegt und das Hosenteil (2) das Kompressionselement (1) und/oder das Taillenband (3) teilweise oder vollständig verdeckt.

12. Kompressionshose nach einem der voranstehenden Ansprüche, wobei das Kompressionselement (1) aus einem elastischen Material, insbesondere einem elastischen Textilmaterial, ist und bei am Patienten angelegter Kompressionshose unter Dehnung des elastischen Materials an dem Taillenband (1) und dem Hosenteil (2) befestigt ist, wodurch das Kompressionselement (1) einen Kompressionsdruck auf den Genitalbereich des Patienten ausübt, wobei der Kompressionsdruck durch Auswahl einer Anlegeposition des Taillenbandes (3) und/oder eines Bundbereiches (2a) des Hosenteils (2) einstellbar ist.

13. Kompressionshose nach einem der voranstehenden Ansprüche, wobei das Hosenteil (2) ein Kompressionstextil aus einem elastischen Textilmaterial ist und insbesondere aus einem elastischen Kompressionsgestrick hergestellt ist, wobei das Textilmaterial bevorzugt so ausgewählt und das Hosenteil so dimensioniert ist, dass das Hosenteil (2), insbesondere im Hüft- und Beckenbereich, einen vorgegebenen Kompressionsdruck auf den Patienten ausübt.

14. Kompressionshose nach einem der voranstehenden Ansprüche, **dadurch gekennzeichnet, dass** das Hosenteil (2) in einem Bundbereich (2a) einen elastischen Bund und/oder einen Tunnelbund aufweist und/oder dass an dem Hosenteil (2) und/oder am Taillenband (3) Hosenträger angeordnet sind.

15. Kompressionshose nach einem der voranstehenden Ansprüche, **gekennzeichnet durch** eine am Kompressionselement (1) lösbar anbringbare oder in eine am Kompressionselement (1) angeformte Tasche einlegbare Pelotte.
